# EUROPEAN PATENT SPECIFICATION

(11) **EP 4 522 274 B1**
(45) Date of publication and mention of the grant of the patent: **04.11.2026**
(21) Application number: 22941822.3
(22) Date of filing: 30.12.2022
(51) Int. Cl.: A61K 33/30, A61P 31/14, C07F 3/06, C07C 337/08

(54) **THIOSEMICARBAZONE COMPLEX, PROCESS OF MANUFACTURE, COMPOSITION AND USE THEREOF IN THE TREATMENT OF COVID-19**
THIOSEMICARBAZONKOMPLEX, VERFAHREN ZUR HERSTELLUNG, ZUSAMMENSETZUNG UND VERWENDUNG DAVON BEI DER BEHANDLUNG VON COVID-1
COMPLEXE DE THIOSEMICARBAZONE, PROCÉDÉ DE FABRICATION, COMPOSITION ET UTILISATION DE CELUI-CI DANS LE TRAITEMENT DU COVID-19

(30) Priority: 09.05.2022 TR 202207486
(43) Date of publication of application: 19.03.2025
(73) Proprietor: T.C. Uskudar Universitesi, 34662 Istanbul (TR); Istanbul Üniversitesi - Cerrahpasa Rektörlügü, Istanbul (TR)
(72) Inventor: ATASEVER ARSLAN, Belkis, Kadikoy/Istanbul (TR); ULKUSEVEN, Bahri, Basaksehir/istanbul (TR); KAYA, Busra, Avcilar/ (TR)
(74) Representative: Sevinç, Cenk
(86) International application number: PCT/TR2022/051727
(87) International publication number: WO 2023/219584

(56) References cited:
- WO-A1-2021/216562
- WO-A1-2021/260409
- KAYA BÜSRA ET AL: "New thiosemicarbazone-based Zinc(II) complexes. In vitro cytotoxicity competing with cisplatin on malignant melanoma A375 cells and its relation to neuraminidase inhibition", vol. 351, 27 November 2021 (2021-11-27), IR, pages 109757, XP093307176, ISSN: 0009-2797, Retrieved from the Internet <URL:https://www.sciencedirect.com/science/article/pii/S0009279721003951?via%3Dihub> DOI: 10.1016/j.cbi.2021.109757
- KAYA BÜSRA ET AL: "Structural characterization of new zinc(ii) complexes with N 2 O 2 chelating thiosemicarbazidato ligands; investigation of the relationship between their DNA interaction and in vitro antiproliferative activity towards human cancer cells", vol. 44, no. 22, 1 January 2020 (2020-01-01), pages 9313 - 9320, XP093307453, ISSN: 1144-0546, Retrieved from the Internet <URL:https://pubs.rsc.org/en/content/articlepdf/2020/nj/d0nj02149k> DOI: 10.1039/D0NJ02149K
- ARSLAN, BELKIS ATASEVER ET AL.: "The iron (III) and nickel (II) complexes with tetradentate thiosemicarbazones. Synthesis, experimental, theoretical characterization, and antiviral effect against SARS-CoV-2", JOURNAL OF MOLECULAR STRUCTURE, vol. 1246, 22 July 2021 (2021-07-22), pages 131166, XP086825980, DOI: 10.1016/j.molstruc.2021.131166
- POLADIAN, QUMARS ET AL.: "A new zinc (II) complex with N202-tetradentate schiff-base derived from pyridoxal-S-methylthiosemicarbazone: Synthesis, characterization, crystal structure, DFT, molecular docking and antioxidant activity studies", POLYHEDRON, vol. 201, 19 March 2021 (2021-03-19), pages 115164, XP086554082, DOI: 10.1016/j.poly.2021.115164

## Description

### Technical Field of the Invention

The invention relates to a thiosemicarbazone complex compound with anti-inflammatory and wound healing properties, which inhibits the 3CL main protease enzyme of SARS-CoV-2 virus and IL-8 cytokine of human bronchial epithelial cells.

### State of the Art

Coronaviruses are a diverse family of viruses that can cause infection in both humans and animals. Several types of coronaviruses can cause mild upper respiratory illness in humans, while viruses such as SARS-CoV and MERS-CoV can cause more serious respiratory illnesses. COVID-19 is an infectious disease caused by severe acute respiratory syndrome coronavirus 2 (SARS-CoV-2). The first case was found in Wuhan city of Hubei province of China, in December 2019. It has continued to spread since then, causing an ongoing pandemic. SARS-CoV-2 infection causes a respiratory disease called COVID-19. Covid-19 shows symptoms in relation to potentially serious complications such as breathing difficulties and pneumonia. SARS-CoV-2, severe acute respiratory syndrome coronavirus 2, is a new type of coronavirus, a positive-sense single-stranded RNA virus. Non-structural proteins (nsp) in the coronavirus-19 genome have a wide range of functions, from transcription of RNA to protein synthesis and modification. Among them, 3CL main protease enzyme is an important target for the development of small inhibitor molecules due to its functional structure and enzyme active sites, and therefore, since 3CL main protease enzyme is an important enzyme related to the replication system of the virus, it is used in inhibition trials in drug studies and has an important place in the fight against the epidemic. The 3CL protease (3CL^{pro}) or parent protease (M^{pro}), formally known as C30 endopeptidase or 3-chymotrypsin benzene protease, found in coronaviruses, cleaves the coronavirus polyprotein at eleven conserved sites. 3CL protease is a cysteine protease. This protease has a cysteine-histidine catalytic dyad at its active site and cleaves a Gln-(Ser/Ala/Gly) peptide bond. The Enzyme Commission refers to this family as the SARS coronavirus major protease (M^{pro}; EC 3.4.2.2.69). 3CL protease corresponds to coronavirus nonstructural protein 5 (nsp5). The "3C" in the common name refers to 3C protease (3C^{pro}), a homologous protease found in picornaviruses. The 3CL^{pro} enzyme, which is an important enzyme associated with the replication system of the virus and used in inhibition trials in drug studies, has an important place in the fight against the epidemic as it is one of the targets of antiviral drugs. In conclusion, 3CL main protease enzyme, also known as 3CL hydrolase enzyme, is an essential enzyme for the maturation of the virus, and therefore studies on the inhibition of 3CL main protease enzyme are carried out in the treatment of C0VID-19 [1],

In the state of the art, another protein that is important in COVID-19 disease is Interleukin-8 (IL-8) cytokine, which is in the category of inflammatory cytokines. IL-8 is a proinflammatory cytokine that has a role in neutrophil activation and has been identified in the pathogenesis and progression of this disease. The increase in IL-8 level causes severe respiratory failure and/or acute respiratory distress syndrome (ARDS) in patients with severe COVID-19. In fact, activation of the immune system and production of inflammatory cytokines are essential for natural antiviral immune responses. However, hyperactivation of the immune system causes an acute increase in circulating pro-inflammatory cytokine levels, leading to a "cytokine storm". Cytokine storm clinically results in systemic inflammation, hyperferritinemia, hemodynamic instability, and multi-organ failure. Tumour necrosis factor alpha (TNF-α), which induces the cytokine IL-8, is important in almost all acute inflammatory reactions and acts as an inflammation promoter. The primary role of TNF is the regulation of immune cells. TNF, an endogenous pyrogen, can trigger fever, apoptotic cell death, cachexia, inflammation, and suppress tumorigenesis and viral replication. TNF-α blockade has been used to treat more than ten different autoimmune inflammatory diseases, suggesting that it could be a potential therapeutic approach to reduce organ damage in COVID-19 patients [2].

In the state of the art, studies on the inhibition of the 3CL main protease enzyme from the enzymes mentioned above have been carried out. In the article of Arslan et al. on tetradentate thiosemicarbazones and iron(III) and nickel(II) complexes, new inhibitors that can be used in the treatment of viral diseases, especially COVID-19, compounds with increased efficiency that inhibit the enzyme and receptor mechanisms are mentioned. This article describes the synthesis of iron(III) and nickel(II) complexes by template condensation of 4-methoxy-salicylaldehyde with 1,1,1-trifluoroacetylacetone (for Fe1) and S-methylthiosemicarbazone derivatives of methylacetoacetate (for Ni1). Complex structures with the N₂O₂-chelating thiosemicarbazidato ligand were identified by analytical, spectroscopic and X-ray crystallography results. The iron(III) centre in the Fe1 complex has a distorted square pyramidal geometry consisting of the N₂O₂ donor set and a chlorine atom, while that of Ni1 is square plane with the set. The inhibitory effect of the synthesized Fe1 compound against the 3CL protease enzyme specific to SARS-CoV-2 virus has been experimentally proven. However, due to their redox activities, iron ions interfere with the vital processes that progress with redox, making a disruptive effect and harming vital processes. In addition, since iron compounds are not easily soluble in water, they accumulate in the tissues and cause chronic toxicity in the body. Thiosemicarbazones mentioned in the study are derivative compounds obtained by condensing an aldehydes or ketone with thiosemicarbazide having the formula H2N-HN-C(=S)-NH2 [3].

Metal-complexed methisazone (zinc or iron) and their use in the treatment of SARS-CoV-2 infections are also described in WO2021/260409A1.

Thiosemicarbazone (TSC) derivatives, which have the structure of Schiff base due to the CH=N- group they contain, are of great interest in pharmacology due to their antiviral, antineoplastic, antimalarial, antifungal and antibacterial properties. It is stated that the biological activities of these compounds may be due to their ability to form chelate complexes with essential heavy metal ions, owing to their being polydentate chelates with two nitrogen atoms and one sulphur atom. Some researchers report that the metal chelates are more active than free ligands and that TSCs and metal complexes obtained with aromatic aldehydes have a strong pharmacological effect. It is stated that the reduction potentials of copper(II)-TSC complexes are directly related to their inhibitory cardiac mitochondrial oxidative phosphorylation and in vitro cytotoxic effects against tumour cells. In a study comparing the spectrophotometric behaviour of various TSC derivatives and copper(II) complexes with superoxide dismutase (SOD), it is emphasized that these compounds show SOD-like activity as well as their broad-spectrum biological activities. Thiosemicarbazones inhibit DNA and RNA synthesis, this effect is due to the fact that thiosemicarbazones are the strongest known inhibitors of ribonucleotide diphosphate reductase, which reduces ribonucleotides to deoxyribonucleotides [4],

Zinc, a heavy metal, is an essential element due to its presence in the structures of certain metallo-enzymes such as cytoplasmic enzymes (superoxide dismutase and phosphodiesterase), mitochondrial enzymes (cytochrome oxidase and pyruvate carboxylase), nuclear enzymes (DNA and RNA polymerase), and Golgi enzymes (peptidase and mannosidase). Zinc, which has extremely low toxicity since it does not accumulate in biological systems, is easily attached to protein chains, and does not damage these chains due to the lack of redox activity. These safe chemical properties of zinc have been one of the reasons why zinc compounds are preferred for drug research. Because of its immunomodulatory properties, zinc is considered to be used as a potential supportive therapy or for the prevention of SARS-CoV-2 infection. In addition, zinc-based antimicrobial materials are widely used in various fields. These materials are generally zinc complexes and ZnO nanoparticles. Many new zinc compounds are synthesized by using Schiff bases or other ligands and the antimicrobial properties of these compounds are investigated. Thiosemicarbazones have antimicrobial, anti-HIV, antiviral, antifungal and anticancer activity. It is also known that they inhibit DNA synthesis by inhibiting the ribonucleotide reductase enzyme. Some zinc(II)-thiosemicarbazone complexes have been reported to have stronger antitumor effects compared to free thiosemicarbazone. It has been shown that zinc(II) complexes obtained from acetylacetone-S-methylthiosemicarbazone have in vitro cytotoxic effects against A375 cancer cells and inhibit the neuraminidase enzyme. In another study, zinc(II) complexes bearing N₂O₂-chelating thiosemicarbazone, especially the zinc(II) complex containing the 4-OCH₃ group, were proposed as an anticancer agent targeting DNA [5],

In its later stages, COVID-19 disease causes high levels of inflammation and thus scarring of BEAS-2B cells, the bronchial epithelial cell line. PHMB is a compound commonly used as a microbicidal agent with good tissue tolerance in compositions used in the state of the art to heal wounds. Wound compositions containing PHMB, applied as gels and lotions, are known. However, there are situations where said compositions cannot provide protection satisfactorily, cannot clean the wounds from harmful substances, do not reduce pain, do not have an ingredient that will support the healing process, and cannot provide moisturising. It has been observed that completely synthetic origin active or auxiliary substances are used in the formulations used in the healing of wounds. Considering that there may be a systemic disease in acute or chronic wounds, taking another synthetic substance may pose an additional risk. Such formulations with synthetic content may cause problems that may adversely affect human health by affecting body functions [6].

In the state of the art, patent no EP2493462B1 relates to a pharmaceutical composition of PTER or its salt, optionally in combination with QUER or any of its salts for preventing and/or treating skin diseases or injuries. As mentioned, it is seen that active or auxiliary substances of synthetic origin are used in the relevant application. It has been observed that completely synthetic origin active or auxiliary substances are used in the formulations used in the healing of wounds. Considering that there may be a systemic disease in acute or chronic wounds, taking another synthetic substance may pose an additional risk. Such formulations with synthetic content may cause problems that may adversely affect human health by affecting body functions.

As the increase in IL-8 due to the fact that the compounds in the state of the art do not inhibit the IL-8 cytokine in human bronchial epithelial cells together with the 3CL main protease enzyme of the SARS-CoV-2 virus causes severe respiratory failure and/or acute respiratory distress syndrome (ARDS) in patients with severe C0VID-19, and causes an acute increase in circulating pro-inflammatory cytokine levels due to hyperactivation of the immune system, leading to a "cytokine storm" resulting in inflammation, hyperferritinemia, hemodynamic instability and multi-organ failure, as these compounds interfere with the life processes progressing with redox and have a disruptive effect and damage them due to the redox activity of the metal ions in these compounds, and as they accumulate in tissues and cause chronic toxicity because they are not easily soluble in water, It has been necessary to make a development in the relevant field.

### Brief Description and Aims of the Invention

In the invention, a thiosemicarbazone complex compound containing zinc(II) ion, which inhibits the 3CL main protease enzyme of SARS-CoV-2 virus, and IL-8 cytokine of human bronchial epithelial cells, is disclosed. The complex compound of the invention is represented by Formula I.

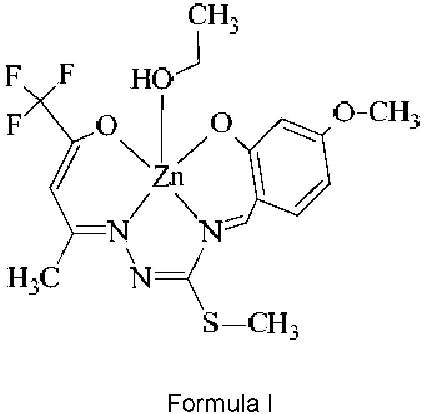

The most important aim of the invention is to reveal a highly effective compound with anti-inflammatory and wound-healing properties in the treatment of COVID-19, which, in short, inhibits the 3CL main protease enzyme, as well as IL-8 cytokine of human bronchial epithelial cells for use in the treatment of COVID-19. Introducing a thiosemicarbazone compound with high therapeutic effects in the treatment of COVID-19 is provided by the synthesis method of said compound.

Another aim of the invention is to provide a compound that inhibits the 3CL main protease enzyme of the SARS-CoV-2 virus. Synthetic compounds containing nitrogen and hydrogen atoms are prone to form hydrogen bonds (bridges) and such compounds are known to be attached to protein chains in this way. In addition, due to the ability of zinc(II) ions to bind to enzyme chains, the zinc centre in the compound strengthens the binding.

Another aim of the invention is to provide a compound that inhibits IL-8 cytokine in human bronchial epithelial cells. In the invention, inhibition of IL-8 cytokine, which is indeed necessary for natural anti-viral immune responses but causes an acute increase in circulating pro-inflammatory cytokine levels with hyperactivation of the immune system, resulting in problems such as systemic inflammation, hyperferritinemia, hemodynamic instability and multi-organ failure, is aimed to be used in the treatment of COVID-19. Inhibition of IL-8 cytokine is provided by the thiosemicarbazone compound of the invention and the method of obtaining this compound.

Another aim of the invention is to provide a compound with anti-inflammatory and wound healing properties. In its later stages, COVID-19 disease causes high levels of inflammation and thus scarring of BEAS-2B cells, the bronchial epithelial cell line. With the complex compound that inhibits the 3CL main protease enzyme as well as the IL-8 cytokine of human bronchial epithelial cells to be used in the treatment of COVID-19, the progression of the COVID-19 disease is prevented, thus preventing the formation of wounds. The subject of the invention is the thiosemicarbazone compound and the method of obtaining this compound to provide a compound with anti-inflammatory and wound healing properties.

Another aim of the invention is to provide an active substance that does not accumulate in tissues and does not cause toxicity to be used in the treatment of COVID-19. The complex compound that is the subject of the invention contains zinc ion as a heavy metal. Zinc ion can easily bind to protein chains such as DNA and enzymes. In addition, unlike other heavy metals such as iron ions, due to the lack of redox activity, it does not interfere with the vital processes progressing with redox and does not have a disruptive effect and does not harm vital processes. Moreover, since zinc compounds are easily soluble in water, they do not accumulate in tissues and do not cause chronic toxicity.

Another aim of the invention is to provide a highly soluble compound for use in the treatment of COVID-19. The binding of the ethyl alcohol molecule (HO-CH₂CH₃) to the zinc ion is a result of the zinc ion's tendency to 5 coordination, increasing the solubility of the complex compound. Ethyl alcohol molecule decomposes in solution medium, test medium or biological system and the effect is due to the zinc(II)-TSC structure that does not contain ethyl alcohol. Ethyl alcohol acts as a means that provides high solubility.

The invention provides a highly soluble thiosemicarbazone complex compound with anti-inflammatory and wound healing properties, that comprises zinc ion, which does not accumulate in tissues and does not cause toxicity and inhibits the 3CL main protease enzyme of SARS-CoV-2 virus, as well as IL-8 cytokine of human bronchial epithelial cells.

### Description of Drawings

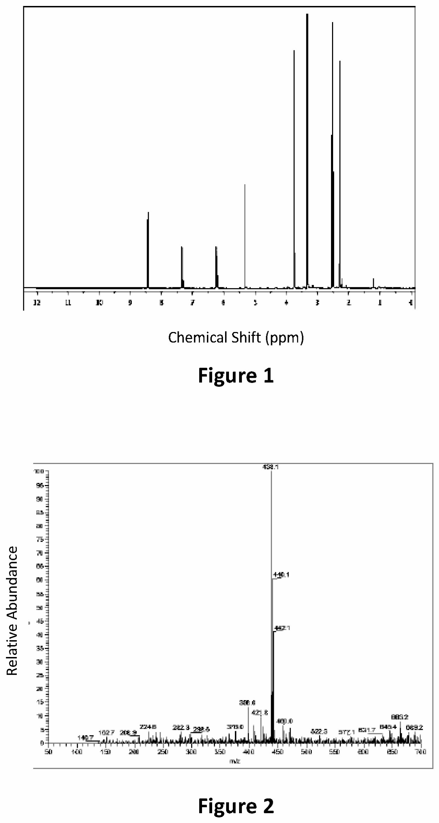

Figure 1. ¹H-NMR spectrum of the compound N-1.1-1,1,1-Trifluoroacetylacetone-N⁴-4-methoxysalicylidene-S-methylthiosemicarbazidato-zinc(II).
Figure 2. Mass spectrum (MS) of the compound N¹-1,1,1-Trifluoroacetylacetone-N⁴-4-methoxysalicylidene-S-methylthiosemicarbazidato-zinc(II).
Figure 3. Graph showing the high inhibitory effect of N¹ -1,1,1-Trifluoroacetylacetone-N⁴-4-methoxysalicylidene-S-methylthiosemicarbazidato-zinc(II).
Figure 4. Graph showing the effect of N¹ -1,1,1-Trifluoroacetylacetone-N⁴-4-methoxysalicylidene-S-methylthiosemicarbazidato-zinc(II) on cell viability.
Figure 5. Graph showing the change in gene expression of N¹-1,1,1-Trifluoroacetylacetone-N⁴-4-methoxysalicylidene-S-methylthiosemicarbazidato-zinc(II).
Figure 6. Microscope images showing healing at 24 and 48 hours after wound formation in N N¹-1,1,1-Trifluoroacetylacetone-N⁴-4-methoxysalicylidene-S-methylthiosemicarbazidato-zinc(II) compound at a concentration of 10 µM.
Figure 7. Graph showing the healing rate at 24 hours after wound formation in N¹-1,1,1-Trifluoroacetylacetone-N⁴-4-methoxysalicylidene-S-methylthiosemicarbazidato-zinc(II) compound at a concentration of 10 µM.
Figure 8. Graph showing the healing rate at 48 hours after wound formation in N¹-1,1,1-Trifluoroacetylacetone-N4-4-methoxysalicylidene-S-methylthiosemicarbazidato-zinc(II) compound at a concentration of 10 µM.

### Detailed Description of the Invention

The invention relates to a thiosemicarbazone complex compound with anti-inflammatory and wound healing properties, which inhibits the 3CL main protease enzyme of SARS-CoV-2 virus and IL-8 cytokine of human bronchial epithelial cells. The complex compound of the invention is represented by Formula I. The chemical name of the thiosemicarbazone-zinc complex compound of the invention is N¹-1,1,1-Trifluoroacetylacetone-N⁴-4-methoxysalicylidene-S-methylthiosemicarbazidato-zinc(II).

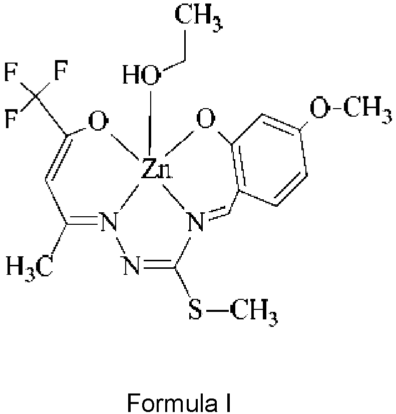

The complex compound that is the subject of the invention is synthesized using 1,1,1-Trifluoroacetylacetone-S-methylthiosemicarbazone hydroiodide, 4-methoxysalicylaldehyde and Zn(CH₃COO)₂.2H₂O compounds. Synthesis method of the compound shown by Formula I, which is the subject of the invention, comprises the process steps of:
i. obtaining Solution 1 by dissolving 0.37 g (1.0 mmol) of thiosemicarbazone derivative, 1,1,1-Trifluoroacetylacetone-S-methylthiosemicarbazone hydroiodide starting material in 5 ml ethyl alcohol in a 25 ml beaker at room temperature,
ii. obtaining Solution 2 by dissolving 0.15 g (1.0 mmol) of 4-methoxysalicylaldehyde in 5 ml of ethyl alcohol in a separate 25 ml beaker at room temperature,
iii. obtaining Solution 3 by adding 0.18 g, 1.0 mmol of Zn(CH₃COO)₂.2H₂O salt in 5 ml of ethyl alcohol to Solution 2,
iv. heating Solution 1 and Solution 3 containers separately to 60°C,
v. mixing Solution 1 and Solution 3 to obtain Solution 4, and stirring Solution 4 at 60°C for 1 hour,
vi. an orange-coloured powdery crystalline solid being formed after about 4 hours after the solution 4 is allowed to cool to room temperature,
vii. Separating the obtained solid by filtration, and
viii. obtaining the complex compound of Formula I by recrystallization of the solid in a 2:1 (v/v) ratio of dichloromethane:ethyl alcohol solvent mixture and drying it in open air for 24 hours.

After 24 hours, the new zinc(II) complex is stored for use in structural analysis and biological activity tests. The synthesis reaction for this process is shown in Reaction 1.

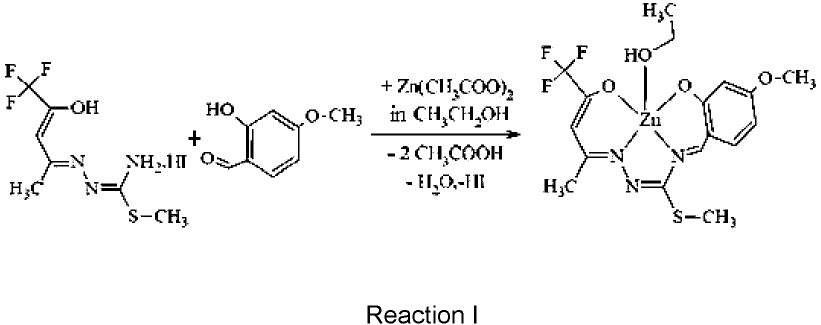

The reason why Zn(CH₃COO)₂.2H₂O salt is first added to 4-methoxysalicylaldehyde in Solution 2 is to prevent the complexation of zinc(II) ions with the thiosemicarbazone derivative in Solution 1 and to preserve the functionality of the template effect in the condensation reaction. The reason for using zinc(II) acetate (Zn(CH₃COO)₂) is to take advantage of the basicity of acetate ions. Thus, there is no need for a base addition to separate the H atom from the -OH groups in the ligand structure.

Characterization analyses of N¹-1,1,1-Trifluoroacetylacetone-N⁴-4-methoxysalicylidene-S-methylthiosemicarbazidato-zinc(II) compound, which is the subject of the invention, were performed by IR, ¹H-NMR and Mass Spectroscopy (MS). IR, ¹H-NMR and MS analysis results of synthesized compounds are shown below.

The molar mass of the compound with the closed formula C₁₇H₁₄ZnF₃N₃O₄S (Formula I) is 484.83 g/mol. The values calculated for the ratios of the elements in this compound are 41.11% C, 4.16% H, 8.67% N, 6.61% S by mass. The values found are 41.68% C, 3.85% H, 8.41% N, 6.98% S by mass.

**IR(cm-1):** v(C=N1) 1607; v(N2=C) 1582; v(N4=C) 1569; v(C-O) 1149, 1128.

**¹H NMR (ppm):** 8.43 (s, 1H, N=CH), 7.34 (d, 1H, c), 6.25-6.20 (m, 2H, a,b), 5.32 (s, 1H, =CH), 4.33 (t, 1H, OH(EtOH)), 3.75 (s, 3H, O-CH₃), 3.45-3.40 (m, 2H, CH₂(EtOH)), 2.52 (s, 3H, S-CH₃), 2.27 (s, 3H, C-CH₃), 1.05 (t, 3H, CH₃(EtOH)).

**+ c ESI MS (m/z, Relative Abundance%):** [M-EtOH+H] 438.1 (100%), 440.1 (60.14%), 442.1 (40.79%).

IR, ¹H-NMR and Mass Spectroscopy (MS) data confirm the structure of the complex compound of the invention. Since bands of NH₂ and OH groups (3354, 3249 and 3042 cm⁻¹) are not observed in S-methylthiosemicarbazone compound, which is the starting compound, in the IR spectrum of the zinc(II) complex compound, this result confirms that the synthesis reaction given above. In the ¹H NMR spectrum, aliphatic and aromatic hydrogen signals are found as expected. In the ¹H NMR spectrum, the hydrogen signals of ethyl alcohol in the 5th coordination site of the central zinc(II) atom are also clearly seen at 4.33, 3.45 and 1.05 ppm values.

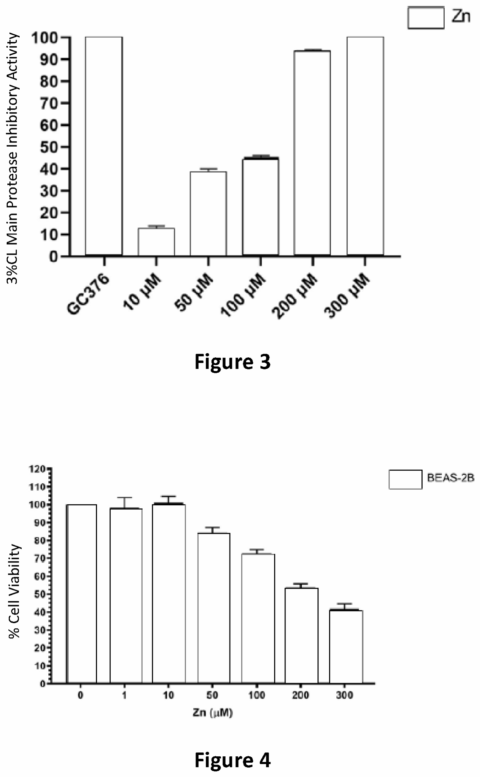

The inhibitory activity of the synthesized N¹-1,1,1-Trifluoroacetylacetone-N⁴-4-methoxysalicylidene-S-methylthio-semicarbazidato-zinc(II) compound, represented by Formula I, against the SARS-Cov-2 3CL enzyme was evaluated with the kit (commercially available BPS Bioscience 3CL Protease assay, Catalogue #79955). The experiment measured how much percent the synthesized compound N¹-1,1,1-Trifluoroacetylacetone-N⁴-4-methoxysalicylidene-S-methylthio-semicarbazidato zinc(II) inhibited the 3CL enzyme at concentrations of 1,10, 25, 50, 100, 200, 300 µM. For the analysis of the inhibitory effect, the inhibitor standard (GC376) included in the kit (BPS Bioscience 3CL Protease assay, Catalogue #79955) was compared with the compound of the present invention. According to the results obtained, it was found that the complex compound had a high inhibitory effect. The results are shown in Figure 3.

In summary, it has been experimentally shown that the complex compound represented by Formula I inhibits the 3CL enzyme (Fig. 3).

The effect of the complex compound represented by the formula I on BEAS-2B cells, the human bronchial epithelial cell line, was investigated. The effect of the synthesized complex compound on cell viability at concentrations of 1,10, 50, 100, 200, 300 µM was measured by the MTT (3-(4,5-dimethylthiazol-2yl)-2,5-diphenyl tetrazolium bromide) method. 10 µl of the substances were added to 96-well plates at a concentration of 1.10, 50, 100, 200, and 300 µM, respectively. Then, 90 µl of BEAS-2B cells prepared with 10⁵ cells/ml were added to the 96-well plate, incubated at 37°C, and the effects of cell viability after 48 hours were examined. After 48 hours incubations, 10 µl of 5 mg/ml MTT in freshly prepared phosphate buffer saline (PBS) was added to the wells and incubated for 3 hours. Dimethyl sulfoxide (DMSO) was added to dissolve the formazan crystals formed by reduction of MTT in living cells. After the solution was kept in the dark for 1 night, it was measured at 570 nm in the spectrometer the next day. The absorbance value of the DMSO control was subtracted from the absorbance of the other wells. Cell viability was calculated by comparison with control cells according to the following equation: $% Cell Viability = \frac{1 - absorbance of the wells incubated with the complex compound}{absorbance of controls} \times 100$

As a result of MTT experiments, it was found that N¹-1,1,1-Trifluoroacetylacetone-N⁴-4-methoxysalicylidene-S-methylthio-semicarbazidato-zinc(II) compound has a proliferative effect at low doses and maintains viability at the rate of 53.69% at 200 µM concentration and 41.76% at 300 µM concentration. The effect of N¹-1,1,1-Trifluoroacetylacetone-N⁴-4-methoxysalicylidene-S-methylthiosemicarbazidato-zinc(II) compound on cell viability is shown in Figure 4. As can be seen, it has been experimentally demonstrated that the compound that is the subject of the present invention has anti-inflammatory and wound-healing effects on human bronchial epithelial cell line BEAS-2B cells, which are one of the cells mainly infected by SARS-Cov-2 virus.

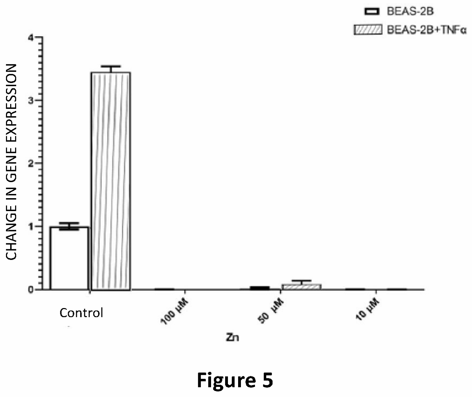

In order to understand the anti-inflammatory effects of the complex compound shown with formula I, an inflammation model was created in BEAS-2B cells with TNFα cytokine. 24 hours after inoculating BEAS-2B cells into 6-well plates at 3x10⁶/3ml, the complex was incubated for 24 hours with concentrations of 10, 50, 100 µM. After incubation with the substances, 5 ng/ml TNF-α was added according to the experimental groups and incubated for 24 hours. After incubation, RNA isolation was performed using the kit (RNeasy Mini Kit of BEAS-2B cells, Qiagen). Complementary DNA (cDNA) was synthesized from 500 ng RNA with the kit (All-In-One 5X RT MasterMix) by measuring the concentrations of the isolated RNAs. IL-8 mRNA gene expression level in BEAS-2B cells incubated with agents and TNF-α was analysed by Real Time Polymerase Chain Reaction (qRT-PCR) method using kit (BrightGreen 2X qPCR MasterMix-ROX). IL-8 mRNA gene expression level was normalized by glyceraldehyde 3-phosphate dehydrogenase (GAPDH). Primer sequences are given in the sequence list as forward primer SEQ ID NO.1 and reverse primer as SEQ ID NO.2. The effect of the complex compound against IL-8 gene expression was examined. It was found to suppress IL-8 gene expression very highly. In addition, Figure 5 shows that when an inflammatory response is generated in BEAS-2B cells with the inflammation model, it suppresses IL-8 gene expression at very high rates.

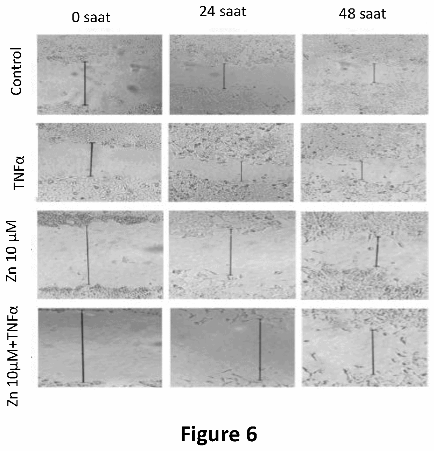

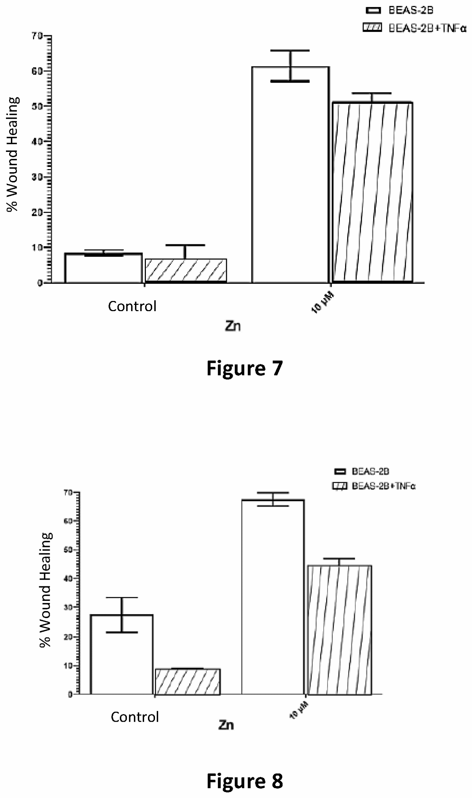

Wound healing test was performed to investigate the wound healing effect of the complex compound shown with formula I in BEAS-2B cells. BEAS-2B cells were inoculated into a 12-well cell culture dish of 10⁶ cells. After 24 hours, the complex was incubated at a concentration of 10 µM for 24 hours. After incubation with the substances, 5 ng/ml TNF-α was added for inflammation and incubated for 24 hours. After approximately 24 hours, a wound was created with a 10 µL micro pipette tip on the surface, which filled the surface of the cell culture container to which they were attached to almost 100%. While creating the wound, the pipette tip was drawn at a constant speed from one end to the other on the cell culture dish in one go. BEAS-2B cells without substance and TNF-α incubation were used as control group. Wound healing activity was determined by visualizing the wound line with phase contrast microscope at 0, 24 and 48 hours and comparing with the control group. At the 10 µM concentration of the complex compound, the healing rate was found to be higher at the 24^{th} and 48^{th} hours after wound formation compared to the control. It was found that there was a higher rate of wound healing effect in the inflammation model induced by TNF α cytokine compared to the control. The improvement rates at 24 and 48 hours are shown in Figure 6 viewed under the microscope and in Figures 7 and 8, respectively.

The complex compound shown with Formula I, which is the subject of the invention, is suitable for use as an inhibitor of 3CL main protease enzyme of SARS-CoV-2 virus and IL-8 cytokine of human bronchial epithelial cells in the treatment of COVID-19 disease caused by SARS-CoV-2 virus. Therefore, a drug containing the complex compound represented by Formula I of the invention is indicated for the treatment of COVID-19 disease caused by the SARS-CoV-2 virus.

### REFERENCES

[1] Dai, W., Zhang, B., Jiang, X. M., Su, H., Li, J., Zhao, Y., Xie, X., Jin, Z., Peng, J., Liu, F., Li, C., Li, Y., Bai, F., Wang, H., Cheng, X., Cen, X., Hu, S., Yang, X., Wang, J.,... Liu, H. (2020). Structure-based design of antiviral drug candidates targeting the SARS-CoV-2 main protease. Science, 368(6497), 1331-1335. https://doi.org/10.1126/science.abb4489
[2] Karki, R., Sharma, B. R., Tuladhar, S., VVilliams, E. P., Zalduondo, L., Samir, P., Zheng, M., Sundaram, B., Banoth, B., Malireddi, R. S., Schreiner, P., Neale, G., Vogel, P., Webby, R., Jonsson, C. B., & Kanneganti, T. D. (2021). Synergism of TNF-a and IFN-y Triggers Inflammatory Cell Death, Tissue Damage, and Mortality in SARS-CoV-2 Infection and Cytokine Shock Syndromes. Cell, 184(1), 149-168.e17. https://doi.org/10.1016/j.cell.2020.11.025
[3] Atasever Arslan, B., Kaya, B., SAhin, O., Baday, S., Saylan, C. C., & Ülküseven, B. (2021). The iron(lll) and nickel(ll) complexes with tetradentate thiosemicarbazones. Synthesis, experimental, theoretical characterization, and antiviral effect against SARS-CoV-2. Journal of Molecular Structure, 1246, 131166. https://doi.org/10.1016/j.molstruc.2021.131166
[4] Campbell, M. J. (1975). Transition metal complexes of thiosemicarbazide and thiosemicarbazones. Coordination Chemistry Reviews, 15(2-3), 279-319. https://doi.org/10.1016/s0010-8545(00)80276-3
[5] Zastrow, M. L., & Pecoraro, V. L. (2014). Designing Hydrolytic Zinc Metalloenzymes. Biochemistry, 53(6), 957-978. https://doi.org/10.1021 /bi4016617
[6] Zhou, Y., Wang, M., Li, Y., Wang, P., Zhao, P., Yang, Z., Wang, S., Zhang, L., Li, Z., Jia, K., Zhong, C., Li, N., Yu, Y., & Hou, J. (2021). SARS-CoV-2 Spike protein enhances ACE2 expression via facilitating Interferon effects in bronchial epithelium. Immunology Letters, 237, 33-41. https://doi.org/10.1016/j.imlet.2021.06.008
[7] Derne 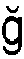 i, A. (2020, April 9). COVID-19 Tedavisinde Kullan 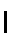 lan İla 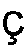 lara Geli 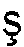 en İstenmeyen İla 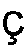 Reaksiyonlari. Türkiye Ulusal Alerji ve Klinik İmmünoloji Derne 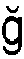 i. https://www.aid.org.tr/COVID-19-tedavisinde-kullanilan-ilaclara-gelisen-istenmeyen-ilac-reaksiyonlari/

## Claims

1. Thiosemicarbazone complex compound with anti-inflammatory and wound healing properties, which inhibits the 3CL main protease enzyme of SARS-CoV-2 virus and IL-8 cytokine of human bronchial epithelial cells, denoted by Formula I:

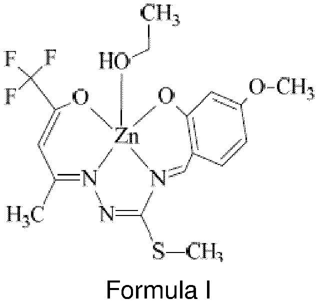

2. A compound according to claim 1 having the formula N¹-1,1,1-Trifluoroacetylacetone-N⁴-4-methoxysalicylidene-S-methylthio-semicarbazidato zinc(II).

3. A compound according to claims 1 or 2, wherein the molar mass of it is 484.83 g/mol.

4. A compound according to claims 1 or 2, comprising 41.68% C, 3.85% H, 8.41% N and 6.98% S by mass.

5. A compound according to claims 1 or 2, wherein the colour of the crystal is orange.

6. A compound according to claims 1 or 2, wherein infrared (IR) spectrum comprises IR (cm-1): v(C=N1) 1607; v(N2=C) 1582; v(N4=C) 1569; v(C-O) 1149, 1128 peaks.

7. A compound according to claims 1 or 2, wherein ¹H-NMR spectrum comprises ¹H NMR (ppm): 8.43 (s, 1H, N=CH), 7.34 (d, 1H, c), 6.25-6.20 (m, 2H, a,b), 5.32 (s, 1H, =CH), 4.33 (t, 1H, OH(EtOH)), 3.75 (s, 3H, O-CH₃), 3.45-3.40 (m, 2H, CH₂(EtOH)), 2.52 (s, 3H, S-CH₃), 2.27 (s, 3H, C-CH₃), 1.05 (t, 3H, CH₃(EtOH)) peaks.

8. A compound according to claims 1 or 2, wherein mass spectrum comprises CESI MS (m/z, Relative Abundance%): [M-EtOH+H] 438.1 (100%), 440.1 (60.14%), 442.1 (40.79%) peaks.

9. Synthesis method of a compound according to claims 1 or 2, comprising the process steps of:
i. obtaining Solution 1 by dissolving 0.37 g (1.0 mmol) of thiosemicarbazone derivative, 1,1,1-Trifluoroacetylacetone-S-methylthiosemicarbazone hydroiodide starting material in 5 ml ethyl alcohol in a 25 ml beaker at room temperature,
ii. obtaining Solution 2 by dissolving 0.15 g (1.0 mmol) of 4-methoxysalicylaldehyde in 5 ml of ethyl alcohol in a separate 25 ml beaker at room temperature,
iii. obtaining Solution 3 by adding 0.18 g, 1.0 mmol of Zn(CH₃COO)₂.2H₂O salt in 5 ml of ethyl alcohol to Solution 2,
iv. heating Solution 1 and Solution 3 containers separately to 60°C,
v. mixing Solution 1 and Solution 3 to obtain Solution 4, and stirring Solution 4 at 60°C for 1 hour,
vi. an orange-coloured powdery crystalline solid being formed after about 4 hours after the solution 4 is allowed to cool to room temperature,
vii. Separating the obtained solid by filtration, and
viii. obtaining the complex compound of Formula I by recrystallization of the solid in a 2:1 (v/v) ratio of dichloromethane:ethyl alcohol solvent mixture and drying it in open air for 24 hours.

10. Anti-inflammatory compound synthesized by the method according to claim 9.

11. Wound healing compound synthesized by the method according to claim 9.

12. Compound that inhibits 3CL main protease enzyme of SARS-CoV-2 virus synthesized by the method according to claim 9.

13. Compound that inhibits IL-8 cytokine in human bronchial epithelial cells synthesized by the method according to claim 9.

14. A pharmaceutical composition comprising a compound according to claims 12 or 13 for use in the treatment of COVID-19 disease.

15. A pharmaceutical composition comprising a compound according to any one of claims 1-8 for use in the treatment of COVID-19 disease.

## Patentansprüche

1. Thiosemicarbazon-Komplexverbindung mit entzündungshemmenden und wundheilenden Eigenschaften, die das 3CL-Hauptproteaseenzym des SARS-CoV-2-Virus und das Zytokin IL-8 in menschlichen Bronchialepithelzellen hemmt, dargestellt durch die Formel I:

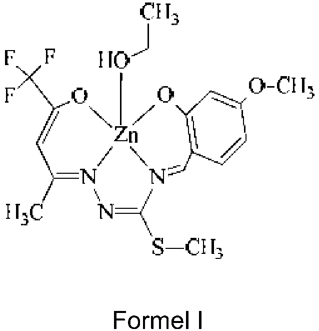

2. Verbindung nach Anspruch 1 mit der Formel N1-1,1,1-Trifluoracetylaceton-N4-4-methoxysalicyliden-S-methylthio-semicarbazidato-Zink(II).

3. Verbindung nach Anspruch 1 oder 2, wobei ihre Molmasse 484,83 g/mol beträgt.

4. Verbindung nach Anspruch 1 oder 2, die 41,68 Massenprozent C, 3,85 Massenprozent H, 8,41 Massenprozent N und 6,98 Massenprozent S enthält.

5. Verbindung nach Anspruch 1 oder 2, wobei die Farbe des Kristalls orange ist.

6. Verbindung nach Anspruch 1 oder 2, wobei das Infrarotspektrum (IR) die folgenden IR-Peaks (cm⁻¹) aufweist: v(C=N1) 1607; v(N2=C) 1582; v(N4=C) 1569; v(C-O) 1149, 1128.

7. Verbindung nach Anspruch 1 oder 2, wobei das 1H-NMR-Spektrum folgende 1H-NMR-Werte (ppm) aufweist: 8,43 (s, 1H, N=CH), 7,34 (d, 1H, c), 6,25-6,20 (m, 2H, a, b), 5,32 (s, 1H, =CH), 4,33 (t, 1H, OH(EtOH)), 3,75 (s, 3H, O-CH3), 3,45-3,40 (m, 2H, CH2(EtOH)), 2,52 (s, 3H, S-CH3), 2,27 (s, 3H, C-CH3), 1,05 (t, 3H, CH3(EtOH)) Peaks.

8. Verbindung nach Anspruch 1 oder 2, wobei das Massenspektrum folgende CESI-MS-Peaks (m/z, relative Häufigkeit in %) umfasst: [M-EtOH+H] 438,1 (100 %), 440,1 (60,14 %), 442,1 (40,79 %).

9. Verfahren zur Herstellung einer Verbindung gemäß Anspruch 1 oder 2, umfassend die folgenden Verfahrensschritte:
i. Herstellung von Lösung 1 durch Auflösen von 0,37 g (1,0 mmol) des Thiosemicarbazon-Derivats, dem Ausgangsstoff 1,1,1-Trifluoracetylaceton-S-methylthiosemicarbazon-Hydroiodid, in 5 ml Ethylalkohol in einem 25-ml-Becherglas bei Raumtemperatur,
ii. Herstellung von Lösung 2 durch Auflösen von 0,15 g (1,0 mmol) 4-Methoxysalicylaldehyd in 5 ml Ethylalkohol in einem separaten 25-ml-Becherglas bei Raumtemperatur,
iii. Herstellung von Lösung 3 durch Zugabe von 0,18 g (1,0 mmol) Zn(CH₃COO)₂·2H₂O-Salz in 5 ml Ethylalkohol zu Lösung 2,
iv. die Behälter mit Lösung 1 und Lösung 3 getrennt auf 60 °C erhitzen,
v. Lösung 1 und Lösung 3 werden gemischt, um Lösung 4 zu erhalten, und Lösung 4 wird 1 Stunde lang bei 60 °C gerührt,
vi. ein orangefarbener, pulverförmiger, kristalliner Feststoff, der sich etwa 4 Stunden nach dem Abkühlen der Lösung 4 auf Raumtemperatur bildet,
vii. Den erhaltenen Feststoff durch Filtration abtrennen und
viii. Gewinnung der komplexen Verbindung der Formel I durch Umkristallisation des Feststoffs in einem Lösungsmittelgemisch aus Dichlormethan und Ethylalkohol im Verhältnis 2:1 (v/v) und anschließende Trocknung an der Luft für 24 Stunden.

10. Entzündungshemmende Verbindung, die nach dem Verfahren nach Anspruch 9 synthetisiert wurde.

11. Wundheilungsmittel, synthetisiert nach dem Verfahren nach Anspruch 9.

12. Verbindung, die das 3CL-Hauptproteaseenzym des SARS-CoV-2-Virus hemmt und nach dem Verfahren nach Anspruch 9 synthetisiert wurde.

13. Verbindung, die das Zytokin IL-8 in menschlichen Bronchialepithelzellen hemmt und nach dem Verfahren nach Anspruch 9 synthetisiert wurde.

14. Pharmazeutische Zusammensetzung, die eine Verbindung nach Anspruch 12 oder 13 enthält, zur Verwendung bei der Behandlung der COVID-19-Erkrankung.

15. Pharmazeutische Zusammensetzung, die eine Verbindung nach einem der Ansprüche 1 bis 8 enthält, zur Verwendung bei der Behandlung der COVID-19-Erkrankung.

## Revendications

1. Composé complexe de thiosémicarbazone présentant des propriétés anti-inflammatoires et cicatrisantes, qui inhibe l'enzyme principale 3CL protéase du virus SARS-CoV-2 et la cytokine IL-8 des cellules épithéliales bronchiques humaines, **caractérisé par** la Formule I :

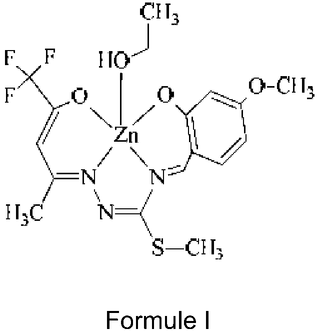

2. Composé selon la revendication 1, ayant pour formule N¹-1,1,1-Trifluoroacétylacétone-N⁴-4-méthoxysalicylidène-S-méthylthiosémicarbazidato de zinc(II).

3. Composé selon la revendication 1 ou 2, dans lequel la masse molaire dudit composé est de 484,83 g/mol.

4. Composé selon la revendication 1 ou 2, comprenant 41,68 % de C, 3,85 % de H, 8,41 % de N et 6,98 % de S en masse.

5. Composé selon la revendication 1 ou 2, dans lequel la couleur du cristal est orange.

6. Composé selon la revendication 1 ou 2, dans lequel le spectre infrarouge (IR) comprend les pics IR (cm-1): v(C=N1) 1607; v(N2=C) 1582; v(N4=C) 1569; v(C-O) 1149, 1128.

7. Composé selon la revendication 1 ou 2, dans lequel le spectre ¹H-NMR comprend les pics ¹H NMR (ppm): 8.43 (s, 1H, N=CH), 7.34 (d, 1H, c), 6.25-6.20 (m, 2H, a,b), 5.32 (s, 1H, =CH), 4.33 (t, 1H, OH(EtOH)), 3.75 (s, 3H, O-CH₃), 3.45-3.40 (m, 2H, CH₂(EtOH)), 2.52 (s, 3H, S-CH₃), 2.27 (s, 3H, C-CH₃), 1.05 (t, 3H, CH₃(EtOH)).

8. Composé selon la revendication 1 ou 2, dans lequel le spectre de masse comprend les pics CESI-MS (m/z, abondance relative %) : [M-EtOH+H] 438,1 (100 %), 440,1 (60,14 %), 442,1 (40,79 %).

9. Procédé de synthèse d'un composé selon la revendication 1 ou 2, comprenant les étapes de procédé suivantes :
i. obtention de la Solution 1 par dissolution de 0,37 g (1,0 mmol) d'un dérivé de thiosémicarbazone, le matériau de départ iodhydrate de 1,1,1-trifluoroacétylacétone-S-méthylthiosémicarbazone, dans 5 ml d'alcool éthylique, dans un bécher de 25 ml, à température ambiante,
ii. obtention de la Solution 2 par dissolution de 0,15 g (1,0 mmol) de 4-méthoxysalicylaldéhyde dans 5 ml d'alcool éthylique dans un bécher séparé de 25 ml à température ambiante,
iii. obtention de la Solution 3 par ajout de 0,18 g (1,0 mmol) de sel Zn(CH₃COO)₂·2H₂O dissous dans 5 ml d'alcool éthylique à la Solution 2,
iv. chauffage séparément des récipients contenant la Solution 1 et la Solution 3 à 60 °C,
v. mélange de la Solution 1 et de la Solution 3 pour obtenir la Solution 4, et agitation de la Solution 4 à 60 °C pendant 1 heure,
vi. formation d'un solide cristallin pulvérulent de couleur orange environ 4 heures après avoir laissé refroidir la Solution 4 à température ambiante,
vii. séparation du solide obtenu par filtration, et
viii. obtention du composé complexe de Formule I par recristallisation du solide dans un mélange de solvants dichlorométhane: alcool éthylique selon un rapport de 2:1 (v/v), puis séchage dudit solide à l'air libre pendant 24 heures.

10. Composé anti-inflammatoire synthétisé par le procédé selon la revendication 9.

11. Composé cicatrisant synthétisé par le procédé selon la revendication 9.

12. Composé inhibant l'enzyme principale 3CL protéase du virus SARS-CoV-2, synthétisé par le procédé selon la revendication 9.

13. Composé inhibant la cytokine IL-8 dans les cellules épithéliales bronchiques humaines, synthétisé par le procédé selon la revendication 9.

14. Composition pharmaceutique comprenant un composé selon la revendication 12 ou 13, destinée à être utilisée dans le traitement de la maladie COVID-19.

15. Composition pharmaceutique comprenant un composé selon l'une quelconque des revendications 1 à 8, destinée à être utilisée dans le traitement de la maladie COVID-19.
